# EUROPÄISCHE PATENTSCHRIFT

(11) **EP 2 224 018 B1**
(45) Veröffentlichungstag und Bekanntmachung des Hinweises auf die Patenterteilung: **30.04.2014**
(21) Anmeldenummer: 09002609.7
(22) Anmeldetag: 25.02.2009
(51) Int. Cl.: C12Q 1/68

(54) **Hybridisierungs- und Detektionspuffer**
Hybridization and detection buffer
Tampon d'hybridation et detection

(43) Veröffentlichungstag der Anmeldung: 01.09.2010
(73) Patentinhaber: QIAGEN GmbH, 40724 Hilden (DE)
(72) Erfinder: Grosshauser, Gerd, 50226 Frechen (DE); Himmelreich, Ralf, 40764 Langenfeld (DE)

(56) Entgegenhaltungen:
- EP-A- 0 167 366
- WO-A-97/43450
- US-B1- 6 361 940
- MASKOS U ET AL: "A STUDY OF OLIGONUCLEOTIDE REASSOCIATION USING LARGE ARRAYS OF OLIGONUCLEOTIDES SYNTHESISED ON A GLASS SUPPORT" NUCLEIC ACIDS RESEARCH, OXFORD UNIVERSITY PRESS, SURREY, GB, Bd. 21, Nr. 20, 1. Januar 1993 (1993-01-01), Seiten 4663-4669, XP000999271 ISSN: 0305-1048
- DEREGT D ET AL: "A multiplex DNA suspension microarray for simultaneous detection and differentiation of classical swine fever virus and other pestiviruses" JOURNAL OF VIROLOGICAL METHODS, ELSEVIER BV, NL, Bd. 136, Nr. 1-2, 1. September 2006 (2006-09-01), Seiten 17-23, XP025030268 ISSN: 0166-0934 [gefunden am 2006-09-01]
- JACOBS K A ET AL: "THE THERMAL STABILITY OF OLIGONUCLEOTIDE DUPLEXES IS SEQUENCE INDEPENDENT IN TETRAALKYLAMMONIUM SALT SOLUTIONS: APPLICATION TO IDENTIFYING RECOMBINANT DNA CLONES" NUCLEIC ACIDS RESEARCH, OXFORD UNIVERSITY PRESS, SURREY, GB, Bd. 16, Nr. 10, 1. Januar 1988 (1988-01-01), Seiten 4637-4650, XP001207450 ISSN: 0305-1048
- MELCHIOR W B ET AL: "ALTERATION OF THE RELATIVE STABILITY OF DA.DT AND DG.DC BASE PAIRS IN DNA" PROCEEDINGS OF THE NATIONAL ACADEMY OF SCIENCES OF THE USA, NEW YORK, NY, US, Bd. 70, Nr. 2, 1. Februar 1973 (1973-02-01), Seiten 298-302, XP002027509

## Beschreibung

Die vorliegende Erfindung betrifft einen wässrigen Hybridisierungs-und/oder Detektionspuffer zum selektiven Nachweis von Nukleotidsequenzen in einer Probe sowie ein Verfahren zum selektiven Nachweis einer oder mehrerer Nukleotidsequenzen in einer Probe.

Einzelsträngige Nukleotidsequenzen besitzen die Fähigkeit, mit komplementären Nukleotidsequenzen über nichtkovalente Bindungen ihrer Nukleobasen Doppelstränge zu bilden (sogenannte Watson-Crick-Basenpaarung). Dieser Vorgang wird als Hybridisierung, die erhaltenen Doppelstränge werden als Hybride bezeichnet. Die Hybridisierung erlaubt einen hochempfindlichen, sequenzspezifischen Nachweis von Oligonukleotidsequenzen und Nukleinsäuren (Polynukleotidsequenzen) und ermöglicht die Identifizierung und Isolierung von Nukleotidsequenzen sowie die Bestimmung des Ausmaßes der Homologie zwischen unterschiedlichen Nukleotidsequenzen. Sie bildet somit die Grundlage für eine Reihe effizienter Techniken der modernen Molekularbiologie wie der quantitativen Genexpressionsanalyse mittels qRT-PCR-Untersuchungen (Echtzeit-Reverse-Transkriptase-Polymerasekettenreaktionen) der mRNA einer Zelle zur Erkennung exprimierter Gene oder der Amplifizierung und Detektion von genetischem Material mittels PCR.

Hierzu wird eine Probe, welche die einzelsträngige Zielnukleotidsequenz enthält, mit einzelsträngigen Oligonukleotiden bekannter Sequenz (Sonden), die (zumindest partiell) zu der Sequenz der nachzuweisenden Zielnukleotidsequenz komplementär sind, unter geeigneten Bedingungen zur Bildung von Hybriden in Kontakt gebracht.

Die Detektion der Hybride erfolgt vorteilhafterweise mit Hilfe eines sogenannten Reportermoleküls, das mit dem Zielnukleotid und/oder der Sonde kovalent oder nicht-kovalent verbunden ist. Durch einen selektiven Nachweis des Reportermoleküls mittels einer geeigneten Detektionsmethode wird auch das mit dem Reportermolekül verbundene Hybrid nachgewiesen. Geeignete Reportermoleküle sind dem Fachmann bekannt und umfassen beispielsweise Fluoreszenzfarbstoffe, Haptene, Enzyme oder radioaktive Atome. Bei einer geeigneten Auswahl dieser Reportermoleküle ist neben der qualitativen auch eine quantitative Bestimmung der Zielnukleotidsequenz in der Probe möglich. Als besonders praktikabel haben sich hierbei Ausführungen erwiesen, bei denen das Zielmolekül oder die Sonde an eine feste Phase gebunden ist. Sogenannte Microarrays von Nukleotidsequenzen, bei denen eine Vielzahl von Oligonukleotidsequenzen als Sonden kovalent an eine zweidimensionale Oberfläche, beispielsweise eine Glasoberfläche, gebunden sind, finden breite Anwendung für analytische und diagnostische Zwecke, da sie unter geeigneten Bedingungen die Hochdurchsatzanalyse einer Vielzahl von Proben über die spezifische Bindung einer Zielnukleotidsequenz an die komplementäre Sonde erlauben. Gegenüber Untersuchungsmethoden in flüssiger Phase bieten festphasenbasierte Methoden die Vorteile größerer Reproduzierbarkeit der Ergebnisse sowie einer höheren Empfindlichkeit und Signalstärke bei der Detektion und ermöglichen die parallele Durchführung einer Vielzahl von Experimenten mit einer geringen Probenmenge in automatisierten Verfahren (siehe bspw. S. Lorkowski et al., Chemie in unserer Zeit, 2000, 34 (6), 356-372).

Eine weitere festphasenbasierte Methode zur Hybridisierung und anschließenden Detektion von Nukleotidsequenzen mit Oligonukleotidsonden basiert auf der Verwendung sogenannter Suspensionsarrays von Mikrosphären (suspension arrays of microspheres/beads). In diesem Verfahren werden Suspensionen von Beads, die im allgemeinen aus organischen Polymeren bestehen, als Festphase verwendet. Durch die interne Markierung der Beads mit unterschiedlichen, genau definierten Konzentrationen zweier verschiedener Fluoreszenzfarbstoffe (sog. Codierung) können beispielsweise einhundert unterschiedliche Gruppen von Beads erzeugt werden, die aufgrund ihrer spezifischen spektralen Erfassbarkeit eindeutig voneinander unterschieden werden können. An jede dieser Gruppen von Beads werden Oligonukleotidsonden einer spezifischen Sequenz gebunden, wobei die Oberfläche der Beads im allgemeinen die Bindung von etwa 10⁸ Sondenmolekülen pro Bead erlaubt. Da jede Gruppen von Beads eine unterschiedlichen Sequenz an ihrer Oberfläche trägt und die einzelnen Gruppen über ihre spektrale Erfassbarkeit unterschieden werden können, können unter Verwendung solcher Suspensionsarrays eine Vielzahl von Zielnukleotiden simultan in einem einzigen Reaktionsgefäß nachgewiesen werden. Vorteilhafterweise wird in diesem Fall als Reportermolekül ein dritter Fluoreszenzfarbstoff verwendet, über den sich das Ausmaß der Reaktion an der Festphasenoberfläche quantifizieren lässt, da die Intensität des gemessenen Signals zur Menge der hybridisierten Probe proportional ist. Derartige Suspensionsarrays sind mittlerweile kommerziell erhältlich; als Beispiel sei auf den ResPlex II Bead Mix der Firma Qiagen (Hilden, Deutschland) zum Nachweis viraler respiratorischer Pathogene verwiesen. Zusätzlich zu den oben bereits erwähnten generellen Vorteilen festphasenbasierter Hybridisierungs- und Detektionstechniken zeichnen sich Festphasentechniken, die auf der Verwendung von Suspensionsarrays beruhen, gegenüber zweidimensionalen Arrays durch eine schnelle Datenakquisition und noch höhere Empfindlichkeit aus und bieten die Möglichkeit, verschiedene Nachweisreaktionen simultan in einer einzigen Probe durchzuführen (sogenannte Multiplexanalysen). Beispielsweise erlaubt das ResPlex II Panel der Firma Qiagen die simultane Amplifizierung und Detektion elf verschiedener viraler Targets aus einer einzigen Probe.

Die Hybridisierung ist eine reversible Reaktion, deren Spezifität und Kinetik (Ausbeute und Reaktionsgeschwindigkeit) von einer Reihe von Faktoren abhängt. Ein Maß für die Spezifität der Hybridisierung unter gegebenen Hybridisierungsbedingungen ist die Stringenz. Je größer die Stringenz der Hybridisierung ist, desto größer ist der Anteil korrekt gepaarter Basenpaare im aus Sonde und Zielsequenz gebildeten Duplex. Als stringente Bedingungen bezeichnet man Hybridisierungsbedingungen, unter denen nur Hybride mit einem hohen Anteil an korrekter Basenpaarung stabil sind. Neben der Temperatur und der Länge der komplementären Nukleotidsequenzen beeinflussen u. a. die Salzkonzentration (bzw. Ionenstärke) sowie die Präsenz organischer Lösungsmittel in der Reaktionslösung und der pH-Wert derselben die Stringenz und auch die Kinetik des Hybridisierungsprozesses. Aus diesem Grund ist das Reaktionsmedium der Hybridisierung, der Hybridisierungspuffer, von besonderer Bedeutung.

Der Hybridisierungspuffer sollte selbstverständlich kompatibel mit der Sonde und dem Zielnukleotid sein, diese also weder durch chemische Reaktion noch in sonstiger Weise beeinflussen oder verändern, ferner eine angemessene Stringenz bei möglichst kurzen Inkubationszeiten bewirken und vorteilhafterweise über einen längeren Zeitraum bei Raumtemperatur lagerfähig sein. Gerade im Hinblick auf die Hochdurchsatzanalyse ist es ferner von Vorteil, wenn in dem Hybridisierungspuffer nicht nur die eigentliche Hybridisierung, sondern darüber hinaus auch der Nachweis (die Detektion) der Hybride erfolgen kann. Dies setzt voraus, dass der Puffer nicht mit dem zur Detektion verwendeten System interferiert. Erfolgt beispielsweise ein Nachweis durch Anfärben der biotinylierten Hybride mit dem Fluoreszenzreporter Streptavidin-(R)-Phycoerythrin (SAPE), so sollte der Puffer weder auf das zur Anfärbung verwendete Protein denaturierend wirken noch die zu detektierende Fluoreszenz des markierten Hybrides beeinflussen.

Neben diesen üblichen Anforderungen an einen Hybridisierungspuffer ergibt sich gerade im Bereich der Multiplexanalyse ferner das Problem, dass neben den oben genannten Faktoren auch die Sequenz der zu untersuchenden Nukleotide die Hybridisierungsrate und die optimale Hybridisierungstemperatur beeinflusst. Während das Guanin(G)-Cytosin(C)-Basenpaar drei Wasserstoffbrückenbindungen auszubilden vermag, kann das Adenin(A)-Thymin(T)/Uracil(U)-Basenpaar lediglich zwei Wasserstoffbrückenbindungen ausbilden. Daher sind G-C-reiche Doppelstrangbereiche thermisch stabiler als A-T-reiche, und die optimale Hybridisierungstemperatur G-C-reicher Doppelstrangbereiche liegt über der A-T-reicher Doppelstrangbereiche gleicher Länge. Während dies bei bestimmten Untersuchungen ein weiteres Spezifitätsmerkmal darstellen kann, erschwert es in Multiplexanalysen die parallele Untersuchung mehrerer unterschiedlicher Sequenzen, sofern deren G-C-Basengehalt nicht vergleichbar oder identisch ist.

Im Stand der Technik werden daher insbesondere für Multiplexsysteme Hybridisierungspuffer verwendet, deren aktiver Hauptbestandteil Tetramethylammoniumionen sind. Hierzu werden im allgemeinen gepufferte Lösungen des wasserlöslichen Tetramethylammoniumchlorids verwendet. Diese Tetramethylammoniumionen-haltigen Puffer erfüllen die oben genannten Anforderungen hinsichtlich der Lagerfähigkeit, der für eine Hybridisierung erforderlichen Inkubationszeiten und sind kompatibel mit einer Vielzahl von Substraten, Reportermolekülen und unterschiedlichen Nachweismethoden. Darüber hinaus ist die Schmelztemperatur von Oligonukleotiddoppelsträngen in Tetraalkylammoniumionen-haltigen Lösungen unabhängig vom G-C-Gehalt einer Nukleotidsequenz, sondern wird lediglich durch deren Länge bestimmt (K. A. Jacobs et al., Nucleic acid research, 1988, 16 (10), 4637-4650; W. I. Wood et al. Proc. Natl. Acad. Sci. USA, 1985, 82, 1585-1588). Aus diesen Gründen werden Tetramethylammoniumionen-haltige Puffer insbesondere für Multiplexanalysen vielfach verwendet. Tetramethylammoniumsalze besitzen allerdings nicht nur einen unangenehm stechenden Geruch, sondern sind darüber hinaus auch giftig (C.-L. Wu et al. J. Occup. Health, 2008, 50, 99-102). EP 0 167 366 offenbart einen Hybridisierungspuffer, der freie NH₄⁺- Konzentrationen von 1M-10M umfaßt.

Die Aufgabe der vorliegenden Erfindung bestand darin, einen Hybridisierungs- und/oder Detektionspuffer bereitzustellen, der einen selektiven Nachweis von Nukleotidsequenzen in einer Probe erlaubt, eine angemessene Stringenz für eine Vielzahl von Substraten in Multiplexanalysen bewirkt und sowohl für die Hybridisierung als auch für die Detektion geeignet ist, darüber hinaus aber keine giftigen und geruchsbelästigenden Substanzen enthält.

Diese Aufgabe wird gelöst durch einen Puffer gemäß Anspruch 1.

Überraschenderweise wurde gefunden, dass ein solcher Puffer auf der Basis von nicht-substituierten Ammoniumionen der Formel NH₄⁺ (im Folgenden als Ammoniumionen bezeichnet) bereitgestellt werden kann, der im Gegensatz zu dem aus dem Stand der Technik bekannten Tetramethylammoniumionen-haltigen Puffer weder giftig noch geruchsbelästigend ist und somit dem Anwender einen gefahrloseren Umgang ermöglicht und darüber hinaus finanzielle Aufwendungen für den Transport und die Lagerung von Gefahrgut erspart.

Der Hybridisierungspuffer bewirkt ferner eine angemessene Stringenz bei möglichst kurzen Inkubationszeiten und ist über einen längeren Zeitraum bei Raumtemperatur lagerfähig. Als kurze Reaktionszeiten werden im Sinne der Erfindung Reaktionszeiten von weniger als drei Stunden, bevorzugt weniger als einer Stunde, besonders bevorzugt weniger als 30 min und insbesondere weniger als 20 min verstanden. Als lagerfähig bei Raumtemperatur wird der erfindungsgemäße Puffer bezeichnet, wenn der Puffer sich nach einer Lagerung bei Raumtemperatur über eine Dauer von einem Monat, bevorzugt von drei Monaten und besonders bevorzugt von einem Jahr weder in seiner chemischen Zusammensetzung noch in seinen physikalischen Eigenschaften verändert. Unter "angemessener" Stringenz werden im Sinne der Erfindung Reaktionsbedingungen verstanden, die der Anforderung an eine hinreichende Spezifität der Hybridisierung unter Berücksichtigung der Reaktionszeit gerecht werden, so dass die Zielnukleotide (Targets) oberhalb der Nachweisegrenze nur an die komplementären Oligonukleotidsonden hybridisieren und diese somit signifikant nachgewiesen werden können.

Darüber hinaus zeichnet sich der erfindungsgemäße Puffer gegenüber dem aus dem Stand der Technik bekannten Puffersystem dadurch aus, dass in dem Testsystem eine höhere Empfindlichkeit in Form einer höheren Signalintensität sowie ein verbessertes Signalrauschverhältnis erzielt werden kann.

Ein Gegenstand der vorliegenden Erfindung ist daher ein wässriger Hybridisierungs- und/oder Detektionspuffer zum selektiven Nachweis von Nukleotidsequenzen in einer Probe umfassend:
1. mindestens eine wasserlösliche Quelle für nicht-substituierte Ammoniumionen der Formel NH₄⁺,
2. mindestens eine Puffersubstanz, die den pH-Wert des Puffers zwischen 6.5 und 9.0 stabilisiert,
3. mindestens einen Chelatbildner,
4. mindestens ein Tensid.
5. optional mindestens einen polymeren Löslichkeitsvermittler.

Mit der Definition Hybridisierungs- und/oder Detektionspuffer ist gemeint, dass der Puffer entweder alleine zur Hybridisierung, alleine zur Detektion, oder sowohl zur Hybridisierung als auch zur Detektion verwendet werden kann.

Als Nukleotidsequenzen werden im Sinne der Erfindung sowohl oligomere als auch polymere Sequenzen von Nukleotiden (Oligonukleotide bzw. Nukleinsäuren), und zwar sowohl von Ribonukleotiden als auch von Desoxyribonukleotiden, bezeichnet. Der Begriff Nukleotidsequenz umfasst im Sinne der Erfindung folglich Oligoribonukleotide, Oligodesoxyribonukleotide, Ribonukleinsäuren (RNA) und Desoxyribonukleinsäuren (DNA). Diese Bezeichnung wird sowohl für die Zielnukleotidsequenz(en) wie auch die Sonde(n) verwendet.

Erfindungsgemäß ist die Ammoniumionenquelle bevorzugt ein Ammoniumsalz der allgemeinen Formel (NH₄)⁺ₙXⁿ⁻, wobei Xⁿ⁻ ein Anion und n eine ganze Zahl zwischen 1 und 3 bedeutet. Das Ammoniumsalz ist bevorzugt ausgewählt aus der Gruppe bestehend aus Ammoniumchlorid, Ammoniumacetat und Ammoniumsulfat, besonders bevorzugt ist Ammoniumchlorid.

Neben Wasser als Lösungsmittel ist das Ammoniumsalz der Hauptbestandteil des erfindungsgemäßen Puffers. Die Konzentration der Ammoniumionen in dem nachfolgend als einfach konzentriert bezeichneten Puffer (1 x Puffer) beträgt bevorzugt 450 - 550 mmol/l, besonders bevorzugt 500 mmol/l. Solch ein 1 x Puffer besitzt eine Dichte von 0.95 - 1.05 g/ml. Unter Verwendung eines geringeren Anteils an Wasser in der Pufferlösung lassen sich entsprechend höher konzentrierte Pufferlösungen darstellen, wobei das molare Verhältnis der übrigen im Puffer enthaltenen Substanzen zu den Ammoniumionen konstant bleibt. So enthält beispielsweise der als dreifach konzentriert bezeichnete Puffer (3 x Puffer) bevorzugt 1.35 - 1.65 mol/l, besonders bevorzugt 1.50 mol/l, der als sechsfach konzentriert bezeichnete Puffer (6 x Puffer) bevorzugt 2.70 - 3.30 mol/l, besonders bevorzugt 3.00 mol/l Ammoniumionen.

Eine Puffersubstanz im Sinne der Erfindung ist ein System aus einer Säure und ihrer konjugierten Base, die den pH-Wert einer Lösung enthaltend diese Puffersubstanzen durch Aufnahme beziehungsweise Abgabe von Protonen, je nach dem, ob in der Lösung durch eine Reaktion oder externe Zugabe Protonen freigesetzt oder verbraucht werden, konstant hält. Die Puffersubstanz im Sinne der Erfindung ist somit zu unterscheiden von der Pufferlösung, nachfolgend kurz Puffer genannt, die neben dem Lösungsmittel und der eigentlichen Puffersubstanz weitere Substanzen, beispielsweise im Falle der vorliegenden Erfindung nicht-substituierte Ammoniumionen, einen oder mehrere Chelatbildner, ein oder mehrere Tenside sowie weitere Substanzen wie Löslichkeitsvermittler, Konservierungsstoffe etc. enthalten kann. Erfindungsgemäß stabilisiert die Puffersubstanz den pH-Wert des Puffers zwischen 6.5 und 9.0, bevorzugt zwischen 6.8 und 8.5 und besonders bevorzugt zwischen 7.5 und 8.5. Bevorzugt im Sinne der Erfindung sind ampholyte Puffersubstanzen, also Verbindungen, die im selben Molekül sowohl mindestens eine saure als auch mindestens eine basische Gruppe tragen und sich somit sowohl sauer als auch basisch verhalten können.

Die Puffersubstanz für den erfindungsgemäßen Puffer ist bevorzugt ausgewählt aus der Gruppe bestehend aus 2-(*N*-Morpholino)methansulfonsäure (MES), 3-(*N*-Morpholino)propansulfonsäure (MOPS), Piperazin-*N*,*N'*-bis(2-ethansulfonsäure) (PIPES), Tris(hydroxymethyl)aminomethanhydrochlorid (TRIS-HCl), *N*-2-Hydroxyethylpiperazinyl-*N*'-2-ethansulfonsäure (HEPES) und *N-*Tris(hydroxymethyl)methylglycin (TRICINE) oder einer Mischung derselben, besonders bevorzugt ist Tris(hydroxymethyl)aminomethanhydrochlorid (TRIS-HCl). Allerdings kann auch jede andere geeignete Puffersubstanz eingesetzt werden, die das Hybridisierungssystem nicht negativ beeinflusst.

In einer bevorzugten Ausführungsform beträgt die Konzentration der Puffersubstanz in dem erfindungsgemäßen Puffer als 1 x Puffer 1 - 100 mmol/l, bevorzugt 2 - 50 mmol/l und besonders bevorzugt 5 - 20 mmol/l.

Der erfindungsgemäße Hybridisierungs- und/oder Detektionspuffer enthält weiterhin bevorzugt mindestens einen Chelatbildner zur Komplexierung zweiwertiger Metallionen, da freie zweiwertige Metallionen aufgrund ihrer Fähigkeiten, Nukleinsäurehybride zu stabilisieren, andernfalls die Stringenz der Hybridisierung und damit die Reproduzierbarkeit der Ergebnisse erheblich beeinflussen können. Bevorzugt ist der Chelatbildner ausgewählt aus der Gruppe bestehend aus Ethylendiamintetraessigsäure (EDTA), *trans*-1,2-Diaminocyclohexan-*N*,*N*,*N*',*N*'-tetraessigsäure (CDTA), Diethylentriaminopentaessigsäure (DTPA), *N-*(2-Hydroxyethyl)ethylendiamin-*N*,*N*',*N*'-triessigsäure (HEDTA), Nitriloessigsäure (NTA), 1,3-Diaminopropan-*N*,*N*,*N*'*,N*'-tetraessigsäure (PDTA) und Triethylentetraaminhexaessigsäure (TTHA) oder eine Mischung derselben, besonders bevorzugt EDTA. In einer bevorzugten Ausführungsform beträgt die Konzentration des Chelatbildners in dem erfindungsgemäßen Puffer als 1 x Puffer 0.01 - 50 mmol/l, bevorzugt 0.1 - 25 mmol/l und besonders bevorzugt 0.5 - 5 mmol/l.

Der erfindungsgemäße Puffer enthält weiterhin bevorzugt mindestens ein Tensid, um die Löslichkeit und Durchmischung des zu untersuchenden Materials mit der Sonde und den Reagenzien in dem Puffer zu verbessern. Bevorzugt im Sinne der Erfindung sind nichtionische und anionische Tenside. Bevorzugt ist das Tensid ausgewählt aus der Gruppe bestehend aus *N*-Acyl-*N-*methylglycinen, wasserlöslichen Salzen von *N*-Acyl-*N*-methylglycinen und Polyoxyethylen-Derivaten von Sorbitanestern oder eine Mischung derselben, besonders bevorzugt im Sinne der Erfindung ist der Puffer ausgewählt aus der Gruppe bestehend aus *N*-Dodecanoyl-*N*-methylglycin, dem Natriumsalz des *N-*Dodecanoyl-*N*-methylglycins (Sarkosyl) und Polyoxyethylen-(20)-sorbitanlaurat (Tween 20). In einer bevorzugten Ausführungsform enthält der erfindungsgemäße Puffer als 1 x Puffer 0.1 - 5 %, bevorzugt 0.4 - 2.5 % des Tensids. Je nachdem, ob das verwendete Tensid bei Raumtemperatur flüssig oder fest ist, beziehen sich die Prozentangaben auf den Gewichtsanteil pro Volumeneinheit (w/v), bspw. bei Sarkosyl, oder den Volumenanteil pro Volumeneinheit (v/v), bspw. bei Tween 20.

Der erfindungsgemäße Puffer kann weiterhin eine oder mehrere Substanzen ausgewählt aus der Gruppe bestehend aus Löslichkeitsvermittlern und Stabilisatoren enthalten, bevorzugt einen polymeren Löslichkeitsvermittler. Geeignete Löslichkeitsvermittler und Stabilisatoren sind dem Fachmann bekannt. Als Löslichkeitsvermittler eignen sich beispielsweise organische Lösungsmittel und/oder Polymere wie beispielsweise Polyoxyalkylenglykole, insbesondere Polyoxyethylenglykole wie PEG 8000. In einer bevorzugten Ausführungsform enthält der erfindungsgemäße Puffer als 1 x Puffer 0.5 - 10 %, bevorzugt 1 - 5 % und besonders bevorzugt 1.5 - 3.0 % (w/v) des Löslichkeitsvermittlers.

Das molare Verhältnis der Ammoniumionen zur Puffersubstanz beträgt bevorzugt 40:1 bis 60:1. Das molare Verhältnis der Ammoniumionen zum Chelatbildner beträgt bevorzugt 400:1 bis 600:1.

In einer bevorzugten Ausführungsform ist der Puffer frei von Tetraalkylammoniumionen R₄N⁺, worin R = Alkyl (CₙH₂ₙ₊₁) (n = 1, 2) darstellt.

Ein weiterer Gegenstand der vorliegenden Erfindung ist die Verwendung des erfindungsgemäßen Puffers zum Nachweis einer Zielnukleotidsequenz in einer Probe durch Hybridisierung mit einer Oligonukleotidsonde, bevorzugt zum simultanen Nachweis mehrerer unterschiedlicher Zielnukleotidsequenzen in einer Probe (Multiplexanalyse). Bevorzugt ist hierbei u. a. die Verwendung zum Nachweis respiratorischer Pathogene, beispielsweise unter Verwendung des Resplex II Panels in Verbindung mit der LiquiChip 200 Workstation der Firma Qiagen sowie alle weiteren auf der QIAplex Technologie (Qiagen, Hilden, Deutschland) basierenden Anwendungen, die auf der Multiplex-Technologie basieren und mit entsprechender Ausrüstung gemessen werden.

Die Erfindung stellt weiterhin ein Verfahren zum selektiven Nachweis einer oder mehrerer unterschiedlicher Nukleotidsequenzen (Zielnukleotidsequenzen) in einer Probe bereit, welches die folgenden Schritte umfasst: 1. Die Hybridisierung der in der Probe enthaltenen Zielnukleotidsequenzen mit komplementären Oligonukleotidsequenzen (Sonden) unter Bedingungen, welche die Hybridisierung komplementärer Nukleotidsequenzen unter Bildung von Hybriden erlauben, durch Vermischen der Zielnukleotidsequenzen und der Sonden in der erfindungsgemäßen Pufferlösung; 2. Umsetzung der erhaltenen Hybride mit einem Nachweisreagenz unter Bildung markierter Hybride; 3. Detektion der markierten Hybride. Die hierzu verwendeten Sonden können natürlichen Ursprungs oder synthetisch hergestellt sein. Bedingungen, welche die Hybridisierung komplementärer Nukleotidsequenzen erlauben, sind dem Fachmann ebenso wie geeignete Nachweisreagenzien und Detektionsverfahren bekannt und werden exemplarisch durch die an späterer Stelle erwähnten Beispiele verdeutlicht.

Bevorzugt wird nicht nur die eigentliche Hybridisierung, sondern auch die Umsetzung der Hybride mit dem Nachweisreagenz und die Detektion der markierten Hybride in dem erfindungsgemäßen Puffer durchgeführt. Hierbei kann zur Hybridisierung der Zielnukleotidsequenzen mit den Sonden und der Umsetzung der Hybride mit dem Nachweisreagenz bevorzugt eine höhere Konzentration an Ammoniumionen verwendet werden als zur Detektion. So kann beispielsweise die Hybridisierung und die Umsetzung der Hybride mit dem Nachweisreagenz in dem als dreifach konzentriert bezeichneten Puffer (3 x Puffer) durchgeführt werden, während das erhaltene Reaktionsgemisch vor der Detektion durch Zugabe von Wasser verdünnt wird, so dass die Konzentration an Ammoniumionen in der zur Detektion verwendeten Lösung bevorzugt 450 - 550 mmol/l beträgt, entsprechend der Konzentration des 1 x Puffers.

Bevorzugt stellt das Nachweisreagenz einen Farbstoff, bevorzugt einen Fluoreszenzfarbstoff, dar. In diesem Falle erfolgt die Detektion der markierten Hybride bevorzugt UV/Vis-spektroskopisch, besonders bevorzugt unter Verwendung von Laser-Quellen.

Bevorzugt können die verwendeten Oligonukleotidsonden an die Oberfläche einer Festphase gebunden sein. Eine bevorzugte Festphase im Sinne der Erfindung sind Beads. Besonders bevorzugt im Sinne der Erfindung ist die Verwendung fluoreszenzkodierter Polystyrolbeads.

Bevorzugt werden in dem erfindungsgemäßen Verfahren biotinylierte Zielsequenzen verwendet. In diesem Fall wird als Nachweisreagenz bevorzugt ein Streptavidin-(R)-Phycoerythrin-Konjugat (SAPE) verwendet.

Bevorzugt im Sinne der Erfindung ist weiterhin ein Verfahren, bei dem die Synthese der biotinylierten Zielnukleotidsequenzen in einem vorhergehenden separaten Schritt mittels Multiplex-PCR unter Verwendung biotinylierter Primer durchgeführt wird. Derartige Primer sind beispielsweise in den kommerziell erhältlichen Kits ResPlex I, ResPlex II und StaphPlex der Firma Qiagen (Hilden, Deutschland) enthalten.

### Beschreibung der Abbildungen

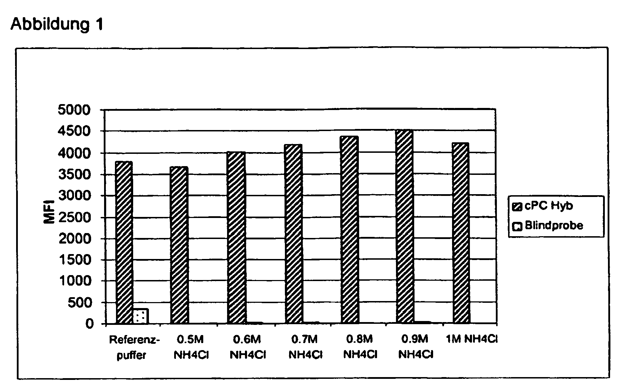

Abbildung 1 zeigt einen Vergleich der Median Fluoreszenzintensität (MFI) SAPE-markierter polystyrolgebundener Hybride (cPC Hyb) in dem bekannten Tetramethylammoniumionen-haltigen Puffer (Referenzpuffer) und in dem erfindungsgemäßen Puffer, der unterschiedliche Konzentrationen an unsubstituierten Ammoniumionen enthält. Zur cPC Hybridisierung (control Probe Complement) wurden am 5'-Ende biotinylierte Zielnukleotidsequenzen mit revers komplementären Sequenzen (Oligonukleotidsonden) umgesetzt, die kovalent an den entsprechenden Beadtyp gekoppelt waren. Zum Vergleich wurde die Umsetzung auch in Abwesenheit des revers komplementären Nukleotids durchgeführt (Blindprobe).

Abbildung 2 zeigt die Anzahl der hybridbildenden Beads (positive counts) in Abhängigkeit von der Ammoniumionenkonzentration des Detektionspuffers im Vergleich mit dem Referenzpuffer.

Abbildung 3 zeigt einen Vergleich des Referenzpuffers mit dem erfindungsgemäßen Puffer in einer Multiplexanalyse zum Nachweis von Phage fr RNA.

Abbildung 4 zeigt eine vergleichende Darstellung der Median Fluoreszenzintensität SAPE-markierter, an Polystyrolbeads gebundener Hybride verschiedener DNA-Zielnukleotidsequenzen, wobei die Hybridisierung und SAPE-Färbung in dem aus dem Stand der Technik bekannten Tetramethylammoniumionen-haltigen Puffer und in verschiedenen Konzentrationen des erfindungsgemäßen Puffers (0.5 M, 1 M, 1.5 M NH₄Cl) durchgeführt wurde.

Abbildung 5 zeigt einen Vergleich der gemessenen Median Fluoreszenzintensität SAPE-markierter, an Polystyrolbeads gebundener Hybride verschiedener DNA-Zielnukleotidsequenzen, wobei die Hybridisierung und SAPE-Färbung in dem aus dem Stand der Technik bekannten Tetramethylammoniumionen-haltigen Puffer (Referenzpuffer = RP) und in dem erfindungsgemäßen Puffer durchgeführt wurde.

Abbildung 6 zeigt einen Vergleich der Anzahl der hybridbildenden Beads in Abhängigkeit von dem verwendeten Hybridisierungspuffer und dem untersuchten Beadtyp.

### Beispiele

Beispiel 1: Herstellung des erfindungsgemäßen Puffers

Zur Herstellung des erfindungsgemäßen Puffers wurden 10 µl einer 1 M wässrigen TRIS-Lösung (pH 8), 5 µl einer 20%-igen (w/v) wässrigen Lösung des Natriumsalzes von *N*-Dodecanoyl-*N*-methylglycin sowie 2 µl einer 0.5 M wässrigen EDTA-Lösung mit den unten spezifizierten Volumina 2 M wässriger Ammoniumchloridlösung versetzt und durchmischt. Der pH-Wert der erhaltenen Lösung betrug pH 8. Zur Herstellung eines Puffers mit einer Ammoniumionenkonzentration von 500 mmol/l (1 x Puffer) wurden 250 µl der 2 M Ammoniumchloridlösung mit den zuvor genannten Substanzen in 733 µl Wasser vermischt. Für eine Ammoniumionenkonzentration von 600 mmol/l wurden entsprechend 300 µl der 2 M Ammoniumchloridlösung und 683 µl Wasser verwendet, für eine Ammoniumionenkonzentration von 700 mmol/l 350 µl der 2 M Ammoniumchloridlösung und 633 µl Wasser, für eine Ammoniumionenkonzentration von 800 mmol/l 400 µl der 2 M Ammoniumchloridlösung und 583 µl Wasser, für eine Ammoniumionenkonzentration von 900 mmol/l 450 µl der 2 M Ammoniumchloridlösung und 533 µl Wasser und für eine Ammoniumionenkonzentration von 1 mol/l (2 x Puffer) 500 µl der 2 M Ammoniumchloridlösung und 483 µl Wasser.

Als Referenzpuffer wurde ein Tetramethylammoniumchlorid-haltiger Puffer folgender Zusammensetzung verwendet: 4.5 M Me₄NCl (Me = Methyl), 0.15% Dodecanoyl-*N*-methylglycin, 75 mM TRIS pH 8, 6 mM EDTA.

Beispiel 2: Hybridisierung revers komplementärer Nukleotidsequenzen

1 µl einer Lösung enthaltend 50 fmol biotinylierter eikosamerer Nukleotide wurde mit 1 µl einer Suspension von Beads, an deren Oberfläche zu den Zielnukleotiden revers komplementäre eikosamere Desoxyribonukleotide (DNA-Sonden) gebunden waren, in jeweils 48 µl der unter Beispiel 1 beschriebenen Puffer mit einer Ammoniumionenkonzentration von 500, 600, 700, 800 und 900 mmol/l sowie 1 mol/l suspendiert. Das erhaltene Gemisch wurde 10 min bei einer Temperatur von 52 °C inkubiert. Anschließend wurde 1 µl 2 X SAPE Lösung der Firma Qiagen (Hilden, Germany; bspw. in den ResPlex I, ResPlex II und StaphPlex Kits enthalten), weitere 5 µl des jeweiligen Hybridisierungspuffers und 4 µl Wasser hinzugegeben, und die Mischung wurde weitere 5 min bei 52 °C inkubiert. Nach Zugabe von weiteren 120 µl des Hybridisierungspuffers wurde die mediane Fluoreszenzintensität und die Anzahl der hybridbildenden Beads (Counts) mit Hilfe der LiquiChip 200 Workstation der Firma Qiagen gemessen.

Zum Vergleich wurde die Hybridisierung auch unter Verwendung des tetramethylammoniumchloridhaltigen Referenzpuffers durchgeführt. Hierzu wurde 1 µl einer Lösung enthaltend 50 fmol biotinylierter eikosamerer Nukleotide mit 1 µl einer Suspension von Beads, an deren Oberfläche zu den Zielnukleotiden revers komplementäre eikosamere Desoxyribonukleotide (DNA-Sonden) gebunden waren, in 39 µl des Referenzpuffers und 9 µl Wasser suspendiert. Das erhaltene Gemisch wurde 10 min bei einer Temperatur von 52 °C inkubiert. Anschließend wurde 1 µl 2 X SAPE Lösung und weitere 9 µl Wasser hinzugegeben, und die Mischung wurde weitere 5 min bei 52 °C inkubiert. Nach Zugabe von weiteren 60 µl des Referenzpuffers wurde die mediane Fluoreszenzintensität und die Anzahl der hybridbildenden Beads (Counts) wie oben beschrieben bestimmt.

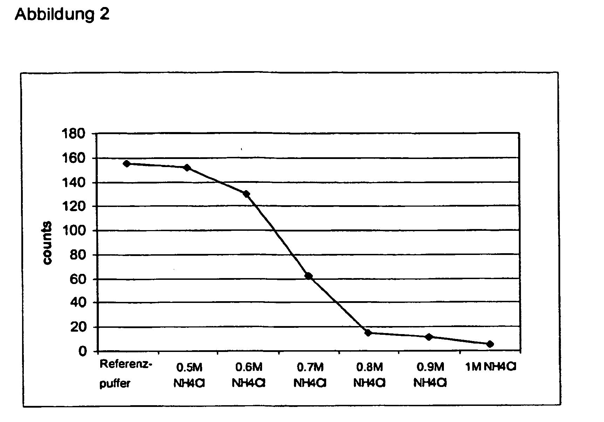

Die graphische Auftragung der Ergebnisse ist in den Abbildungen 1 und 2 dargestellt. Aus diesen Abbildungen ist zu erkennen, dass mit zunehmender Ammoniumionenkonzentration die gemessene mediane Fluoreszenzintensität zunahm. Allerdings sank ab einer Ammoniumchloridkonzentration von etwa 0.6 mol/l die Anzahl der hybridbildenden Beads. Es ist daher vorteilhaft, zur Detektion eine Ammoniumionenkonzentration von etwa 500 mmol/l zu verwenden. Bei dieser Konzentration weist der erfindungsgemäße Puffer eine Dichte von ungefähr 1 g/ml auf. Des Weiteren ist zu erkennen, dass die gemessene mediane Fluoreszenzintensität bei Verwendung des Ammoniumionen-haltigen Puffers vergleichbar ist zu derjenigen, die in dem Tetramethylammoniumionen-haltigen Referenzpuffer gemessenen wurde.

Zur Bestimmung der Selektivität wurde die Umsetzung der funktionalisierten Beads mit dem SAPE-Konjugat unter den oben beschriebenen Bedingungen auch in Abwesenheit des biotinylierten revers komplementären Nukleotids durchgeführt (Blindprobe). Die Ergebnisse dieser Messungen sind in Abbildung 1 als gepunktete Balken dargestellt. Überraschenderweise ist das Ausmaß der unspezifischen Reaktion, verursacht durch die Bindung des SAPE-Konjugats an nicht-hybridisierte Beads, im Ammoniumionen-haltigen Puffer deutlich geringer als im Tetramethylammoniumionen-haltigen Puffer.

Beispiel 3: RT-PCR-Amplifizierung von Phage fr RNA mit anschließender Hybridisierung und Detektion der amplifizierten viralen RNA.

In einer RT-PCR Amplifizierung von Phage fr RNA gemäß Qiagen ResPlex II Handbuch 03/2007 wurden in Gegenwart des ResPlex II Primergemisches die biotinylierten Zielnukleotide amplifiziert. Zur Überprüfung der Spezifität der Hybridisierung und Nachweisreaktion wurde das erhaltene PCR-Gemisch anschließend mit dem kommerziell erhältlichen ResPlex II Bead Mix der Firma Qiagen in Kontakt gebracht, welcher eine Mischung von Bead-gebundenen Oligonukleotidsonden zum Nachweis der folgenden viralen respiratorischen Pathogene enthält: respiratorische synzytische Viren (RSVA, RSVB), Influenza A und B Viren (INFAA, INFB), Parainfluenza-Viren (PIV 1, PIV 2, PIV 3, PIV 4), humaner Metapneumovirus (hMPV), Coxsackieviren/Echovirus (CVEV) und Rhinovirus (RHV).

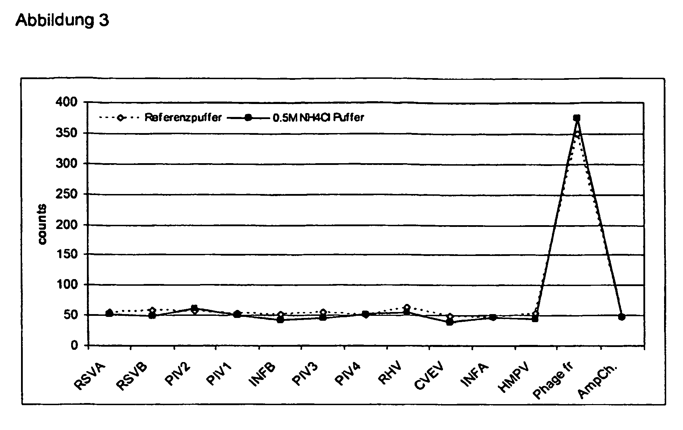

Zur Hybridisierung wurde der 2 x Hybridisierungspuffer, enthaltend 1 mol/l NH₄Cl, 20 mmol/l TRIS pH 8, 0.2 % Dodecanoyl-*N*-methylglycin und 2 mmol/l EDTA verwendet. Zu einem Gemisch aus 25 µl des 2 x Hybridisierungspuffers, 1 µl der Phage fr spezifisch markierten Beads (enthaltend etwa 1000 Beads pro µl, hergestellt gemäß allgemeiner Vorgaben, siehe http://www1.qiagen.com/literature/handbooks/literature.aspx?id=1000123) und 1 µl ResPlex II bead mix (Qiagen, enthaltend etwa 300 beads pro Bead Code und µl) in 21 µl Wasser wurden 2 µl der aus der RT-PCR-Reaktion erhaltenen Lösung gegeben, welche die biotinylierten amplifizierten Zielnukleotide enthielt. Die resultierende Mischung wurde 10 min bei 52 °C inkubiert. Zur Markierung der Hybride wurden 5 µl des 2 x SAPE-Puffers sowie weitere 5 µl des Hybridisierungspuffers hinzugefügt, und die resultierende Mischung wurde weitere 5 min bei 52 °C inkubiert. Nach Zugabe von weiteren 120 µl Hybridisierungspuffer wurde die mediane Fluoreszenzintensität für die unterschiedliche Sonden tragenden Beads sowie die Anzahl der validen hybridbildenden Beads unter Verwendung der LiquiChip 200 Workstation der Firma Qiagen bestimmt. Zum Vergleich wurde die oben beschriebene Reaktionssequenz auch in dem Tetramethylammoniumionen-haltigen Referenzpuffer anstelle des Ammoniumionen-haltigen Puffers durchgeführt. Die Ergebnisse sind in Tabelle 1 und Abbildung 3 dargestellt.

**Tabelle 1**

| **Puffer** | **Anzahl der zur RT-PCR eingesetzten Kopien** | **RSVA** | **RSVB** | **PIV2** | **PIV1** | **INFB** | **PIV3** | **PIV4** | **RHV** | **CVEV** | **INFA** | **HMPV** | **Phage fr** | **AmpCh.** |
|---|---|---|---|---|---|---|---|---|---|---|---|---|---|---|
| Bead Code | | 013 (13) | 014 (14) | 026 (26) | 027 (27) | 028 (28) | 029 (29) | 031 (31) | 035 (35) | 036 (36) | 037 (37) | 044 (44) | 046 (46) | 052 (52) |
| Referenzpuffer | 10⁴ | 19 | 23 | 25 | 42 | 35 | 45.5 | 25.5 | 6.5 | 0 | 133.5 | 16 | 1112 | 31 |
| Referenzpuffer | 10³ | 20 | 4 | 27 | 21 | 32 | 23.5 | 19 | 15 | 10 | 72 | 25 | 159 | 31 |
| Referenzpuffer | 10² | 9.5 | 14 | 7 | 22 | 23 | 31 | 21 | 15 | 1 | 46 | 35.5 | 137 | 28.5 |
| Referenzpuffer | 10¹ | 22 | 19 | 23 | 16.5 | 10 | 14.5 | 25 | 33 | 27.5 | 67 | 1 | 74 | 12.5 |
| 0.5 M NH₄Cl Puffer | 10⁴ | 17.5 | 12 | 14 | 21 | 11 | 18 | 15 | 21.5 | 21 | 118 | 7 | 1049 | 37 |
| 0.5 M NH₄Cl Puffer | 10³ | 0 | 30.5 | 10 | 22 | 24 | 21 | 25 | 32 | 14 | 94 | 0 | 171 | 33 |
| 0.5 M NH₄Cl Puffer | 10² | 24 | 20 | 6.5 | 0 | 17 | 48 | 2.5 | 34 | 20.5 | 92 | 0 | 138.5 | 5 |
| 0.5 M NH₄Cl Puffer | 10¹ | 13.5 | 18.5 | 11 | 188 | 53 | 14 | 9 | 14.5 | 22.5 | 121.5 | 9.5 | 78 | 8 |

Tabelle 1 zeigt die gemessene mediane Fluoreszenzintensität der unterschiedlichen Gruppen von Mikrosphären in Abhängigkeit von der ursprünglich in RT-PCR eingesetzten Anzahl an Kopien der Phage fr RNA und des zur Hybridisierung und SAPE-Färbung verwendeten Puffers. Es ist zu erkennen, dass im Rahmen der statistischen Schwankungen für beide Puffer vergleichbare Ergebnisse erzielt werden. Der erfindungsgemäße Puffer kann folglich den bekannten Tetramethylammoniumionen-haltigen Puffer auch in Multiplexanalysen zum Nachweis respiratorischer Pathogene unter Verwendung des ResPlex II Panels und der LiquiChip 200 Workstation ersetzen, ohne dass eine Änderung der bereits für den Tetramethylammoniumionen-haltigen Puffer bestehenden Protokolle nötig wäre. Ferner ist zu erkennen, dass auch bei einer sehr geringen Anzahl an Kopien (10²), die ursprünglich zur RT-PCR eingesetzt wurden, noch ein spezifischer Nachweis der amplifizierten RNA erfolgen kann.

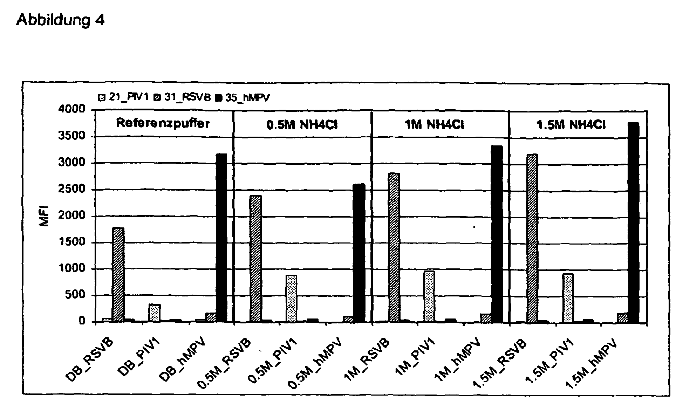

Die Anzahl der in dem Ammoniumionen-haltigen Puffer detektierten hybridbildenden Beads für Phage fr lag sogar geringfügig höher als die in dem Tetramethylammoniumionen-haltigen Puffer, wie aus Abbildung 4 ersichtlich ist.

Beispiel 4: Hybridisierung und Detektion von PCR-Produkten

Da die Hybridisierung bei höherer Ammoniumionenkonzentration einerseits zwar stärkere mediane Fluoreszenzintensitäten zeigte, andererseits die Anzahl an hybridbildenden Beads etwas zurückging (vergleiche Beispiel 1), wurde im Folgenden die Hybridisierung und SAPE-Färbung bei einer Ammoniumionenkonzentration von über 0.5 mol/l durchgeführt und die Konzentration dieser Lösung durch Verdünnung vor der Messung auf eine Konzentration an Ammoniumionen von 0.5 mol/l eingestellt.

Hierzu wurden analog zu Beispiel 1 doppelt (2 x), vierfach (4 x) und sechsfach (6 x) konzentrierte Pufferlösungen hergestellt. Die Zusammensetzung dieser Lösung ist in Tabelle 2 wiedergegeben.

**Tabelle 2:**

| **2 x Puffer** | **4 x Puffer** | **6 x Puffer** |
|---|---|---|
| 1 M NH₄ Cl | 2 M NH₄ Cl | 3 M NH₄ Cl |
| 20 mM TRIS pH 8 | 40 mM TRIS pH 8 | 60 mM TRIS pH 8 |
| 0.66% (v/v) Tween 20 | 1.33% (v/v) Tween 20 | 2% (v/v) Tween 20 |
| 5.33% (w/v) PEG 8000 | 10.66% (w/v) PEG 8000 | 16% (w/v) PEG 8000 |
| 2 mM EDTA | 4 mM EDTA | 6 mM EDTA |

Unter Verwendung von Plasmiden als Templates wurden 3 PCRs entsprechend des Qiagen ResPlex II, Handbuch 03/2007 durchgeführt. Da Plasmide der zu amplifizierenden Fragmente benutzt wurden, wurde hierbei auf den Schritt der reversen Transkription verzichtet.

Zu einer Mischung von jeweils 1 µl des ResPlex Bead Mix II (jeweils etwa 300 Beads pro Code und µl) in 17 µl Wasser und 25 µl des jeweiligen Hybridisierungspuffers (2 x, 4 x oder 6 x) wurden 5 µl der erhaltenen PCR gegeben. Die Mischungen wurde 10 min bei 52 °C inkubiert und anschließend mit 5 µl 2x-SAPE-Lösung und 5 µl des jeweiligen Hybridisierungspuffers versetzt. Anschließend wurden die resultierenden Mischungen erneut 5 min bei 52 °C inkubiert. Vor der Detektion wurden die Mischungen auf eine Konzentration an Ammoniumionen von 0.5 mol/l verdünnt. Im Falle des 2 x Puffers erfolgte dies durch Zugabe von 120 µl 0.5 x Hybridisierungspuffer, im Falle des 4 x Puffers durch Zugabe von 120 µl 0.25 x Puffers und im Falle des 6 x Puffers durch Zugabe von 120 µl Wasser. Zum Vergleich wurde die Reaktion auch in dem Tetramethylammoniumionen-haltigen Referenzpuffer durchgeführt.

Die Ergebnisse sind in Abbildung 4 dargestellt. Zunächst einmal ist zu erkennen, dass bei jeder verwendeten Konzentration an Ammoniumionen im erfindungsgemäßen Puffer eine ähnliche Verteilung an unspezifischen Signalen zu beobachten ist wie bei dem Tetramethylammoniumionen-haltigen Referenzpuffer. Weiterhin ist zu erkennen, dass die mediane Fluoreszenzintensität ansteigt, wenn zur Hybridisierung und SAPE-Färbung ein erfindungsgemäßer Puffer höherer Ammoniumionenkonzentration verwendet wird, obwohl die Detektion nach Verdünnung vorgenommen wurde und alle Ansätze dann die gleiche Konzentration an Ammoniumionen aufwiesen. Es ist also vorteilhaft, die Hybridisierung und SAPE-Färbung in einem konzentrierten Puffer (Ammoniumionengehalt > 0.5 mol/l) durchzuführen und den Ansatz vor der Messung auf einer Ammoniumionenkonzentration von 0.5 mol/l zu verdünnen.

Beispiel 5: Vergleichende Multiplexanalyse respiratorischer RNA-Pathogene

Die gemäß Beispiel 4 erhaltenen PCR-Lösungen wurden in einer Multiplexanalyse mit dem kommerziell erhältlichen ResPlex II Bead Mix der Firma Qiagen zum simultanen Nachweis respiratorischer RNA-Pathogene umgesetzt.

Zu 5 µl der unter Beispiel 4 beschriebenen PCR-Lösungen wurden 25 µl des 6 x Hybridisierungspuffers und 15 µl Wasser gegeben. Die Mischung wurde 10 min bei 52 °C inkubiert, danach wurden 5 µl 2 x SAPE-Puffer und weitere 5 µl 6 x Hybridisierungspuffer hinzugegeben, und die resultierende Mischung wurde weitere 5 min bei 52 °C inkubiert. Vor der Messung wurde der Ansatz durch Zugabe von 120 µl Wasser auf eine Ammoniumionenkonzentration von 0.5 mol/l verdünnt. Die mediane Fluoreszenzintensität der unterschiedlichen Beads sowie die Anzahl der hybridbildenden Beads wurden mit Hilfe der Liquichip 200 Workstation der Firma Qiagen gemessen. Zum Zwecke des Vergleiches wurden die Reaktion und die Messung auch in dem Tetramethylammoniumionen-haltigen Referenzpuffer durchgeführt.

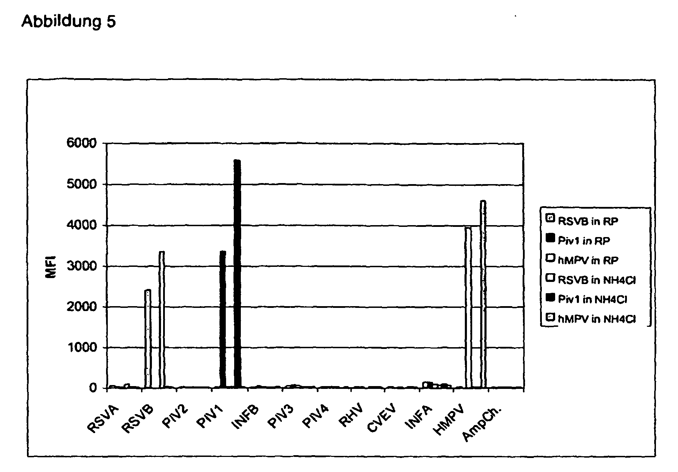

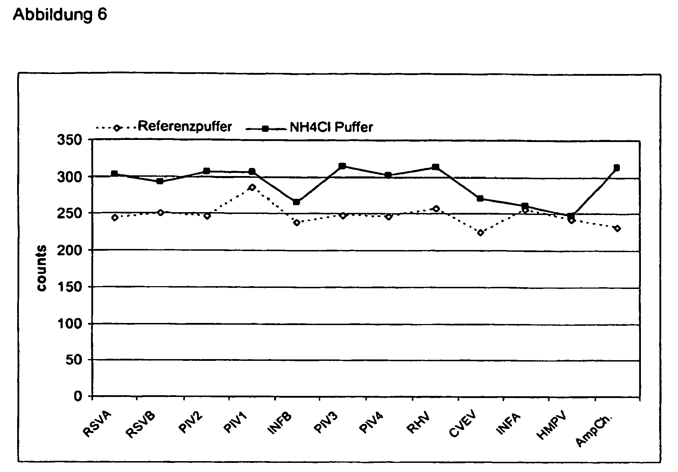

Die Ergebnisse dieser Untersuchungen sind in Abbildung 5 und 6 dargestellt. Aus Abbildung 5 ist zu erkennen, dass sich der erfindungsgemäße Puffer gegenüber dem bekannten Tetramethylammoniumionen-haltigen Puffer durch eine höhere mediane Fluoreszenzintensität sowie ein besseres Signal-Rausch-Verhältnis auszeichnet. Ferner liegt auch die detektierte Anzahl der hybridbildenden Beads in dem erfindungsgemäßen Puffer über der in dem Tetramethylammoniumionen-haltigen Puffer, wie aus Abbildung 6 ersichtlich ist.

## Patentansprüche

1. Wässriger Puffer zum selektiven Nachweis von Nukleotidsequenzen in einer Probe umfassend
(1) mindestens eine wasserlösliche Quelle für nicht-substituierte Ammoniumionen der Formel NH₄⁺, wobei die Konzentration der Ammoniumionen 450-550 mmol/l beträgt,
(2) mindestens eine Puffersubstanz, die den pH-Wert des Puffers zwischen 6.5 und 9.0 stabilisiert,
(3) mindestens einen Chelatbildner,
(4) mindestens ein Tensid,
(5) optional mindestens einen polymeren Löslichkeitsvermittler.

2. Puffer gemäß Anspruch 1, wobei die Ammoniumionenquelle ein Ammoniumsalz der allgemeinen Formel (NH₄)⁺ₙ Xⁿ⁻ darstellt, worin Xⁿ⁻ ein Anion und n eine ganze Zahl zwischen 1 und 3 bedeutet, bevorzugt ein Ammoniumsalz ausgewählt aus der Gruppe bestehend aus Ammoniumchlorid, Ammoniumacetat und Ammoniumsulfat, besonders bevorzugt Ammoniumchlorid.

3. Puffer gemäß Anspruch 1 oder 2, wobei die Puffersubstanz ausgewählt ist aus der Gruppe bestehend aus 2-(*N*-Morpholino)ethansulfonsäure (MES), 3-(*N-*Morpholino)propansulfonsäure (MOPS), Piperazin-*N,N'*-bis(2-ethansulfonsäure) (PIPES), Tris(hydroxymethyl)aminomethanhydrochlorid (TRIS-HCl), *N*-2-Hydroxyethylpiperazinyl-*N'*-2-ethansulfonsäure (HEPES) und *N*-Tris(hydroxymethyl)methylglycin (TRICINE) oder eine Mischung derselben, bevorzugt Tris(hydroxymethyl)aminomethanhydrochlorid (TRIS-HCl).

4. Puffersubstanz gemäß irgendeinem der Ansprüche 1 bis 3, wobei der Chelatbildner ausgewählt ist aus der Gruppe bestehend aus Ethylendiamintetraessigsäure (EDTA), *trans*-1,2-Diaminocyclohexan-*N,N,N',N'*-tetraessigsäure (CDTA), Diethylentriaminopentaessigsäure (DTPA), *N*-(2-Hydroxyethyl)ethylendiamin-*N,N,N'*-triessigsäure (HEDTA), Nitrilotriessigsäure (NTA), 1,3-Diaminopropan-*N,N,N',N'*-tetraessigsäure (PDTA) und Triethylentetraaminhexaessigsäure (TTHA) oder eine Mischung derselben, bevorzugt EDTA.

5. Puffer gemäß irgendeinem der Ansprüche 1 bis 4, wobei das Tensid ausgewählt ist aus der Gruppe bestehend aus *N*-Acyl-*N*-methylglycinen, wasserlöslichen Salzen von *N*-Acyl-*N*-methylglycinen und Polyoxyethylen-Derivaten von Sorbitanestern oder eine Mischung derselben, besonders bevorzugt ausgewählt aus der Gruppe bestehend aus *N*-Dodecanoyl-N methylglycin, dem Natriumsalz des *N*-Dodecanoyl-*N*-methylglycins und Polyoxyethylen-(20)-sorbitanlaurat.

6. Puffer gemäß irgendeinem der Ansprüche 1 bis 5, wobei der Puffer weiterhin eine oder mehrere Substanzen ausgewählt aus der Gruppe bestehend aus Löslichkeitsvermittlern und Stabilisatoren enthält.

7. Puffer gemäß irgendeinem der Ansprüche 1 bis 6, wobei das molare Verhältnis der Ammoniumionen zur Puffersubstanz 40 : 1 bis 60 : 1 und das molare Verhältnis der Ammoniumionen zum Chelatbildner 400 : 1 bis 600 : 1 ist.

8. Puffer gemäß irgendeinem der Ansprüche 1 bis 7, wobei der Puffer frei von Tetraalkylammoniumionen ist.

9. Verwendung eines Puffers gemäß irgendeinem der Ansprüche 1 bis 8 zum Nachweis einer Zielnukleotidsequenz in einer Probe durch Hybridisierung mit einer Oligonukleotidsonde.

10. Verwendung gemäß Anspruch 9 zum simultanen Nachweis mehrerer Zielnukleotidsequenzen in einer Probe.

11. Verfahren zum selektiven Nachweis einer oder mehrerer Nukleotidsequenzen (Zielnukleotidsequenzen) in einer Probe umfassend die folgenden Schritte:
(1) Hybridisierung der in der Probe enthaltenen Zielnukleofidsequenzen mit komplementären Oligonukleotidsequenzen (Sonden) unter Bedingungen, welche die Hybridisierung komplementärer Nukleotidsequenzen unter Bildung von Hybriden erlauben, durch Vermischen der Zielnukleotidsequenzen und der Sonden in einer Pufferlösung gemäß irgendeinem der Ansprüche 1 bis 8,
(2) Umsetzung der erhaltenen Hybride mit einem Nachweisreagenz unter Bildung markierter Hybride,
(3) Detektion der markierten Hybride.

12. Verfahren gemäß Anspruch 11, wobei auch die Umsetzung der Hybride mit dem Nachweisreagenz und die Detektion der markierten Hybride in einem Puffer gemäß irgendeinem der Ansprüche 1 bis 8 durchgeführt werden.

13. Verfahren gemäß Anspruch 11 oder 12, wobei das Nachweisreagenz einen Farbstoff, bevorzugt einen Fluoreszenzfarbstoff darstellt und die Detektion UV/Vis-spektroskopisch, bevorzugt unter Verwendung von Laser-Quellen erfolgt.

14. Verfahren gemäß irgendeinem der Ansprüche 11 bis 13, wobei die Oligonukleotidsonden an die Oberfläche einer Festphase, bevorzugt an Beads, gebunden sind.

15. Verfahren gemäß Anspruch 14, wobei als Festphase fluoreszenzkodierte Polystyrolbeads verwendet werden.

16. Verfahren gemäß irgendeinem der Ansprüche 11 bis 15, wobei die Zielnukleotidsequenzen biotinyliert sind und als Nachweisreagenz bevorzugt ein Streptavidin-(R)-Phycoerythrin-Konjugat (SAPE) verwendet wird.

17. Verfahren gemäß Anspruch 16, umfassend einen Schritt zur Herstellung der biotinylierten Zelnukleotidsequenzen mittels Multiplex-PCR unter Verwendung biotinylierter Primer.

18. Verfahren gemäß einem der Ansprüche 11 bis 17, wobei das System aus Schritt (1) vor Schritt (3) durch Zugabe von Wasser oder einem Puffer niedrigerer Konzentration als in Schritt (1) verwendet, verdünnt wird.

## Claims

1. Aqueous buffer for the selective detection of nucleotide sequences in a sample, comprising
(1) at least one water-soluble source of non-substituted ammonium ions of the formula NH₄⁺, wherein the concentration of the ammonium ions is 450-550 mmol/l,
(2) at least one buffer substance which stabilizes the pH of the buffer between 6.5 and 9.0,
(3) at least one chelating agent,
(4) at least one surfactant,
(5) optionally at least one polymeric solubilizer.

2. Buffer according to Claim 1, wherein the source of ammonium ions is an ammonium salt of the general formula (NH₄)⁺ₙXⁿ⁻, where Xⁿ⁻ is an anion and n is an integer between 1 and 3, preferably an ammonium salt selected from the group consisting of ammonium chloride, ammonium acetate and ammonium sulphate, particularly preferably ammonium chloride.

3. Buffer according to Claim 1 or 2, wherein the buffer substance is selected from the group consisting of 2-(N-morpholino)ethanesulphonic acid (MES), 3-(N-morpholino)propanesulphonic acid (MOPS), piperazine-N,N'-bis(2-ethanesulphonic acid) (PIPES), tris(hydroxymethyl)aminomethane hydrochloride (Tris-HCl), N-2-hydroxyethylpiperazinyl-N'-2-ethanesulphonic acid (HEPES) and N-tris(hydroxymethyl)methylglycine (TRICINE) or a mixture of the same, preferably tris(hydroxymethyl)aminomethane hydrochloride (Tris-HCl).

4. Buffer substance according to any of Claims 1 to 3, wherein the chelating agent is selected from the group consisting of ethylenediaminetetraacetic acid (EDTA), *trans*-1,2-diaminocyclohexane-N,N,N',N'-tetraacetic acid (CDTA), diethylenetriaminopentaacetic acid (DTPA), N-(2-hydroxyethyl)ethylenediamine-N,N',N'-triacetic acid (HEDTA), nitrilotriacetic acid (NTA), 1,3-diaminopropane-N,N,N',N'-tetraacetic acid (PDTA) and triethylenetetraaminehexaacetic acid (TTHA) or a mixture of the same, preferably EDTA.

5. Buffer according to any of claims 1 to 4, wherein the surfactant is selected from the group consisting of N-acyl-N-methylglycines, water-soluble salts of N-acyl-N-methylglycines and polyoxyethylene derivatives of sorbitan esters or a mixture of the same, particularly preferably selected from the group consisting of N-dodecanoyl-N-methylglycine, the sodium salt of N-dodecanoyl-N-methylglycine and polyoxyethylene (20) sorbitan laurate.

6. Buffer according to any of Claims 1 to 5, wherein the buffer further comprises one or more substances selected from the group consisting of solubilizers and stabilizers.

7. Buffer according to any of Claims 1 to 6, wherein the molar ratio of the ammonium ions to the buffer substance is 40:1 to 60:1 and the molar ratio of the ammonium ions to the chelating agent is 400:1 to 600:1.

8. Buffer according to any of Claims 1 to 7, wherein the buffer is free from tetraalkylammonium ions.

9. Use of a buffer according to any of Claims 1 to 8 for detecting a target nucleotide sequence in a sample by hybridization with an oligonucleotide probe.

10. Use according to Claim 9 for the simultaneous detection of multiple target nucleotide sequences in a sample.

11. Method for the selective detection of one or more nucleotide sequences (target nucleotide sequences) in a sample comprising the following steps:
(1) hybridization of the target nucleotide sequences present in the sample with complementary oligonucleotide sequences (probes), under conditions which allow the hybridization of complementary nucleotide sequences, to form hybrids by mixing the target nucleotide sequences and the probes in a buffer solution according to any of Claims 1 to 8,
(2) reaction of the hybrids obtained with a detection reagent to form labelled hybrids,
(3) detection of the labelled hybrids.

12. Method according to Claim 11, wherein the reaction of the hybrids with the detection reagent and the detection of the labelled hybrids are also carried out in a buffer according to any of Claims 1 to 8.

13. Method according to Claim 11 or 12, wherein the detection reagent constitutes a dye, preferably a fluorescent dye, and the detection is carried out by UV/Vis spectroscopy, preferably using laser sources.

14. Method according to any of Claims 11 to 13, wherein the oligonucleotide probes are bound to the surface of a solid phase, preferably to beads.

15. Method according to Claim 14, wherein the solid phase used is fluorescent coded polystyrene beads.

16. Method according to any of Claims 11 to 15, wherein the target nucleotide sequences are biotinylated and the detection reagent used is preferably a streptavidin-(R)-phycoerythrin conjugate (SAPE).

17. Method according to Claim 16, comprising a step for preparing the biotinylated target nucleotide sequences by means of multiplex PCR using biotinylated primers.

18. Method according to any of Claims 11 to 17, wherein the system from step (1) is diluted before step (3) by addition of water or a buffer of lower concentration than that used in step (1).

## Revendications

1. Tampon aqueux destiné à la détection sélective de séquences de nucléotides dans un échantillon, comprenant
(1) au moins une source hydrosoluble d'ions ammonium non substitués de formule NH₄⁺, la concentration des ions ammonium étant de 450-550 mmoles/l,
(2) au moins une substance tampon qui stabilise le pH du tampon entre 6,5 et 9,0,
(3) au moins un agent chélateur,
(4) au moins un tensioactif,
(5) en option au moins un tiers-solvant polymère.

2. Tampon selon la revendication 1, dans lequel la source d'ions ammonium représente un sel d'ammonium de formule générale (NH₄)⁺ₙXⁿ⁻, dans laquelle Xⁿ⁻ représente un anion et n un nombre entier compris entre 1 et 3, de préférence un sel d'ammonium choisi dans le groupe constitué par le chlorure d'ammonium, l'acétate d'ammonium et le sulfate d'ammonium, de façon particulièrement préférée le chlorure d'ammonium.

3. Tampon selon la revendication 1 ou 2, dans lequel la substance tampon est choisie dans le groupe constitué par l'acide 2-(*N*-morpholino)éthanesulfonique (MES), l'acide 3-(*N*-morpholino)propanesulfonique (MOPS), l'acide pipérazine-*N,N'*-bis(2-éthanesulfonique (PIPES), le chlorhydrate de tris(hydroxyméthyl)amino-méthane (TRIS-HCl), l'acide *N*-2-hydroxyéthyl-pipérazinyl-*N'*-2-éthanesulfonique (HEPES) et la *N*-tris-(hydroxyméthyl)méthylglycine (TRICINE) ou un mélange de ceux-ci, de préférence est le chlorhydrate de tris(hydroxyméthyl)aminométhane (TRIS-HCl).

4. Substance tampon selon l'une quelconque des revendications 1 à 3, dans laquelle l'agent chélateur est choisi dans le groupe constitué par l'acide éthylènediaminetétraacétique (EDTA) l'acide *trans-*1,2-diaminocyclohexane-*N,N,N',N'*-tétraacétique (CDTA), l'acide diéthylènetriaminopentaacétique (DTPA), l'acide *N*-(2-hydroxyéthyl)éthylènediamine-*N,N',N'*-triacétique (HEDTA), l'acide nitrilotriacétique (NTA), l'acide 1,3-diaminopropane-*N,N,N',N'*-tétraacétique (PDTA) et l'acide triéthylènetétraaminehexaacétique (TTHA) ou un mélange de ceux-ci, de préférence est l'EDTA.

5. Tampon selon l'une quelconque des revendications 1 à 4, dans lequel le tensioactif est choisi dans le groupe constitué par des *N*-acyl-*N-*méthylglycines, des sels hydrosolubles de *N*-acyl-*N-*méthylglycines et des dérivés polyoxyéthyléniques d'esters de sorbitanne ou un mélange de ceux-ci, de façon particulièrement préférée choisi dans le groupe constitué par la *N*-dodécanoyl-*N*-méthylglycine, le sel de sodium de la *N*-dodécanoyl-*N*-méthylglycine et le laurate polyoxyéthylénique (20) de sorbitanne.

6. Tampon selon l'une quelconque des revendications 1 à 5, le tampon contenant en outre une ou plusieurs substances choisies dans le groupe constitué par des tiers-solvants et des stabilisants.

7. Tampon selon l'une quelconque des revendications 1 à 6, dans lequel le rapport molaire des ions ammonium à la substance tampon vaut de 40 : 1 à 60 : 1 et le rapport molaire des ions ammonium à l'agent chélateur vaut de 400 : 1 à 600 : 1.

8. Tampon selon l'une quelconque des revendications 1 à 7, le tampon étant exempt d'ions tétraalkylammonium.

9. Utilisation d'un tampon selon l'une quelconque des revendications 1 à 8, pour la détection d'une séquence de nucléotides cible dans un échantillon, par hybridation avec une sonde oligonucléotidique.

10. Utilisation selon la revendication 9, pour la détection simultanée de plusieurs séquences de nucléotides cibles dans un échantillon.

11. Procédé pour la détection sélective d'une ou de plusieurs séquences de nucléotides (séquences de nucléotides cibles) dans un échantillon, comprenant les étapes suivantes :
(1) hybridation des séquences de nucléotides contenues dans l'échantillon, avec des séquences oligonucléotidiques complémentaires (sondes) dans des conditions qui permettent l'hybridation de séquences de nucléotides complémentaires avec formation d'hybrides, par mélange des séquences de nucléotides cibles et des sondes dans une solution tampon selon l'une quelconque des revendications 1 à 8,
(2) mise en réaction des hybrides obtenus avec un réactif de détection, avec formation d'hybrides marqués,
(3) détection des hybrides marqués.

12. Procédé selon la revendication 11, dans lequel on effectue la réaction des hybrides avec le réactif de détection et la détection des hybrides marqués également dans un tampon selon l'une quelconque des revendications 1 à 8.

13. Procédé selon la revendication 11 ou 12, dans lequel le réactif de détection représente un colorant, de préférence un colorant fluorescent, et la détection s'effectue par spectroscopie UV/Vis, de préférence avec utilisation de sources laser.

14. Procédé selon l'une quelconque des revendications 11 à 13, dans lequel les sondes oligonucléotidiques sont fixées à la surface d'une phase solide, de préférence à des billes.

15. Procédé selon la revendication 14, dans lequel on utilise comme phase solide des billes en polystyrène codées par fluorescence.

16. Procédé selon l'une quelconque des revendications 11 à 15, dans lequel les séquences de nucléotides cibles sont biotinylées et on utilise comme réactif de détection de préférence un conjugué streptavidine-(R)-phycoérythrine (SAPE).

17. Procédé selon la revendication 16, comprenant une étape pour la production des séquences de nucléotides cibles biotinylées, par PCR-multiplex avec utilisation d'amorces biotinylées.

18. Procédé selon l'une quelconque des revendications 11 à 17, dans lequel avant l'étape (3) on dilue le système provenant de l'étape (1), par addition d'eau ou d'un tampon à plus faible concentration que celui utilisé dans l'étape (1).
